# EUROPEAN PATENT APPLICATION

(11) **EP 4 527 225 A1**
(43) Date of publication of application: **26.03.2025**
(21) Application number: 23823314.2
(22) Date of filing: 11.05.2023
(51) Int. Cl.: A41B 11/00, A41B 11/02, A63B 71/02, A63B 71/12

(54) **PROPULSIVE ELASTIC STOCKING**

(30) Priority: 15.06.2022 ES 202231006 U
(71) Applicant: Modesto Martin Gonzalez, S.L, 46650 Canals, Valencia (ES)
(72) Inventor: MARTIN GONZALEZ, Modesto, 46650 CANALS, Valencia (ES); BENEYTO ABAD, Francisco José, 03700 DENIA, Alicante (ES)
(74) Representative: Sahuquillo Huerta, Jesús
(86) International application number: PCT/ES2023/070302
(87) International publication number: WO 2023/242452

(57) **Abstract**

The invention relates to a propulsive elastic sock that allows different muscles and tendons, such as the gastrocnemius muscle, the soleus muscle or the Achilles tendon, to be protected and supported during different sports practices. The sock comprises a first fabric (1), a second fabric (2), an elastic cuff (3) and a toe (4) and is characterized in that the fabric (1) with greater elastic force is located vertically or diagonally on the rear part, the more lightweight fabric (2) is located on the front part, the elastic cuff (3) is located at the upper end, and the toe (4) is located at the lower end.

## Description

### Technical field

The objective of the present invention is to create a propelling elastic sock that can provide protection and support for various muscles and tendons, including the twin muscles, the soleus, and the Achilles tendon, during the performance of different sports practices.

### Background Art

At present, a number of solutions exist for the protection of the leg and foot during the performance of a variety of sports activities.

Among the existing solutions is the international application WO2008110640, which describes a sports garment with a reinforced fabric zone. This zone, or zones, comprises at least one additional layer of terry-type fabric. The curl-like fabric is arranged in a snack-type formation on a base fabric layer or layers of the sock.

In contrast, the Spanish invention ES0086029U represents an advancement in the field of sports apparel. It is distinguished by its construction as a single piece of knitted fabric, encompassing the foot and the leg, such that the foot is integrated with the rest of the garment. The floor is resistant and flexible, formed as a sole of footwear. The assembly is complemented in the part of the foot with buttresses of the heel, a toe box, and a template of the same configuration as that of the sole, in the manner of a footwear sole.

In general, these solutions aim to safeguard the foot and leg by means of compression with tissues of an appropriate nature, with the objective of addressing the various issues and injuries that may arise during the performance of specific sports activities. However, none of the aforementioned solutions are designed to prevent such lesions and to enhance sports performance, as is the case with the elastic sock presented herein.

### Description of the invention

The technical problem that this invention addresses is the need to provide protection and support to the muscles and tendons in the foot and leg area during the performance of various sporting activities. To this end, the elastic propulsion sock, the subject of this utility model, comprises a fabric with greater elastic force placed vertically or diagonally at the back, a lighter fabric at the front, an elastic cuff at its upper end, and a toe at its lower end. The sock's design features a vertical division from the leg to the middle of the foot, with the fabric exhibiting the greatest elastic force positioned at the back. This configuration offers protection for muscles such as the gastrocnemius or soleus and the Achilles tendon. In this region, the sock's properties elicit vertical actions analogous to the eccentric and concentric contractions of the gastrocnemius and soleus muscles, as well as the elastic extension and contraction of the Achilles tendon.

In the anterior tibial region and on the instep, the fabric is lighter in weight to provide support for the remaining contours. These fabrics exhibit markedly disparate elastic, tensile, and densitometric characteristics. The addition of analogous elastic materials to the exterior and interior of the fabric may be undertaken with the objective of enhancing the fabric's functionality. Additionally, the elastic cuff at the top enables the sock to fit the leg correctly, while the toe provides adequate protection for the toes. The recommended sock confers numerous advantages to the user, irrespective of their athletic level. These include reduced muscular fatigue and enhanced strength, enabling prolonged exercise intensity; an extended stride length; and an improved running pace, with a performance enhancement of up to 3%. The results demonstrated a 6% improvement in the prevention of discomfort and overloads in the soleus, gastrocnemius, and Achilles tendon when the sock was used, as well as an improvement in muscle reactivation after performing any type of activity.

### Brief description of the figures

The following is a concise overview of several illustrations, with the objective of enhancing comprehension of the invention. These illustrations are explicitly related to a specific embodiment of the invention and are presented as a non-limiting example.

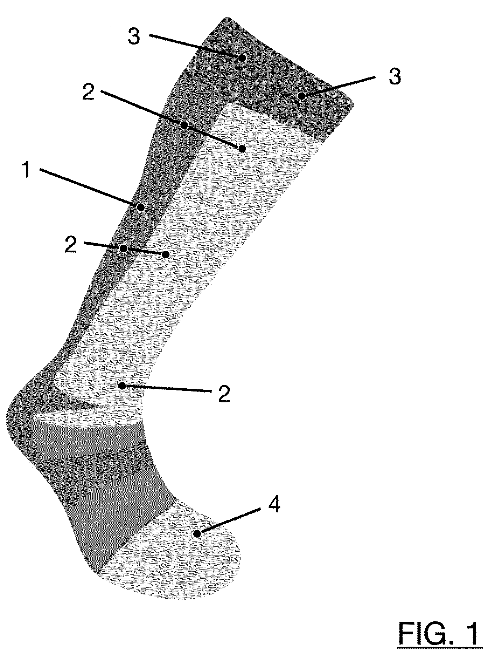

FIG 1. It shows an overview of the elastic propulsion sock, the subject of this utility model.

### Detailed explanation of the embodiments

The figures that follow illustrate a preferred embodiment of the invention. In particular, the elastic propulsion sock comprises a first fabric (1) with greater elastic force, which is placed vertically or diagonally at the back. The second fabric (2), which is lighter in weight, is located at the front. An elastic cuff (3) is located at the upper end of the sock, while a toe (4) is located at the lower end. The fabrics (1) and (2) can incorporate one or more elastic bands arranged vertically.

In a preferred embodiment, the fabric (1) located at the back is composed of coated elastane and pinched elastane, or another material with equivalent elastic properties. In a second preferred embodiment, the fabric (2) located at the front is composed of coated elastane and polyamide, or another material with equivalent elastic properties. In a third preferred embodiment, the elastic cuff (3) is composed of polyamide and coated elastane or another material with equivalent elastic properties. In a fourth preferred embodiment, the toe is constructed from cotton or an alternative material with analogous elastic properties.

In the initial practical implementation, in which a user performs the act of running with an elastic propulsion sock, it can be observed that in the moment prior to the push-off to take a stride, the foot is located behind the vertical line of the hip in dorsiflexion (flexed foot). Consequently, the propulsive elastic sock is stretched at the back (1), loaded with elastic energy and ready to contract. Subsequently, upon the commencement of the concentric contraction of the corresponding muscles (soleus and gastrocnemius), the release of the elastic energy stored in the sock itself coincides with the initiation of the push-off phase. Consequently, the user attains augmented energy for the aforementioned push-off and the same stride power with diminished muscular and tendon exertion.

In a second practical implementation, in which a user performs a jump with an elastic propulsion sock, it can be observed that prior to any push-off, the user flexes their knees and ankles in order to load the muscles with energy. This allows for all the joints to be extended from this flexed position, thus enabling the jump to be performed. In the moment preceding the jump, when the ankle is flexed, the propulsive elastic sock is loaded with elastic energy at the back (1) when all its elements are stretched. This energy is released at the precise moment the jump is performed, when the elastic elements contract, thereby providing greater power to the jump.

Subsequently, the user must flex their knees and ankles to cushion the weight of the body during the falling phase after a jump. This moment, when the muscles engage in eccentric contraction to support the weight and prevent a fall, is followed by the contact of the user's foot with the ground and the subsequent flexion of the ankle. At this point, the back of the propulsive elastic sock (1) provides external assistance to the muscles, thereby reducing the exertion of the eccentric contraction phase and allowing the muscles to become less fatigued by cushioning the impact of the jump.

## Claims

1. Elastic propulsion sock comprising a first fabric (1), a second fabric (2), an elastic cuff (3) and a toe (4) **characterised by the fact that** the fabric (1) with the greater elastic force is placed vertically or diagonally at the back, the lighter fabric (2) is located at the front, the elastic cuff (3) is located at its upper end and the toe (4) at its lower end.

2. Elastic propulsion sock according to claim 1, where fabric (1) and fabric (2) can incorporate one or more elastic bands arranged vertically.

3. Elastic propulsion sock according to claim 1, where the fabric (1) located at the back is made of coated elastane and pinched elastane.

4. Elastic propulsion sock according to claim 1, where the fabric (2) located in the front is made of coated elastane and polyamide.

5. Elastic propulsion sock according to claim 1, where the elastic cuff (3) is made of polyamide and coated elastane.

6. Elastic propulsion sock according to claim 1, where the toe (4) is made of cotton.
